# EUROPEAN PATENT SPECIFICATION

(11) **EP 3 208 332 B1**
(45) Date of publication and mention of the grant of the patent: **04.03.2020**
(21) Application number: 15850818.4
(22) Date of filing: 16.10.2015
(51) Int. Cl.: C12N 9/38, A23C 9/152, C12N 9/96

(54) **LACTASE SOLUTION AND MILK USING SAME**
LAKTASELÖSUNG UND MILCH DAMIT
SOLUTION DE LACTASE ET LAIT L'UTILISANT

(30) Priority: 17.10.2014 JP 2014213093
(43) Date of publication of application: 23.08.2017
(73) Proprietor: Godo Shusei Co., Ltd., Matsudo-shi, Chiba 271-0064 (JP)
(72) Inventor: NAGAHATA, Naoki, Matsudo-shi Chiba 271-0064 (JP); HORIGUCHI, Hirofumi, Matsudo-shi Chiba 271-0064 (JP)
(74) Representative: Hoffmann Eitle
(86) International application number: PCT/JP2015/079259
(87) International publication number: WO 2016/060224

(56) References cited:
- EP-A1- 0 074 237
- EP-A1- 1 208 848
- JP-A- H 034 791
- JP-A- H01 117 786
- JP-A- S59 104 556
- JP-A- 2004 534 527

## Description

### Technical Field

The present invention relates to a lactase solution containing reducing sugar in a predetermined value or less.

### Background Art

Lactase is an enzyme that degrades lactose into glucose and galactose. Lactose is present in a dairy product produced by using a milk raw material such as milk. Lactase is present in many of the human small intestines, therefore, lactose in a dairy product is decomposed into glucose and galactose in the small intestine. However, in some humans in which lactase does not act sufficiently, lactose is not sufficiently decomposed, and symptoms of diarrhea and dyspepsia are caused. In order to prevent such symptoms, lactase has been widely used to decompose the lactose in advance to produce dairy products.

In the current technical level, it is extremely difficult to chemically synthesize lactase, therefore, lactase to be used for the production of dairy products is produced using microorganisms such as yeast, mold, and bacteria. However, the microorganisms also produce substances other than the lactase, therefore, substances other than lactase are generally contained in a lactase solution.

Herein, there is a drawback that in general, enzymes have low stability against heat, pH changes, and the like, and further are susceptible to the influence of coexisting substances. In particular, in a case where the form of containing enzymes is a system containing water or an aqueous solution, there is a problem that the activity is gradually lowered also during the storage. As to the deactivation of enzymes, in general, the denaturation deactivation based on the structural changes due to thermal motion is considered. In addition, in a case where a proteolytic enzyme is contained as a substance other than the lactase, deactivation due to mutual degradation of enzymes (self-digestion) is also generated. In order to solve these problems, measures such as the addition of various stabilizers, and the refrigerated storage are taken.

For example, in order to reduce the deactivation by the action of an antiseptic agent, there is a technique in which sugars are put into a glycerol kinase solution (Patent Literature 1). In addition, it has been proposed to add polysaccharides to stabilize a protease solution (Patent Literature 2).

### Citation List

### Patent Literature

Patent Literature 1: JP 2004-141162 A
Patent Literature 2: JP 3-4790 A

### Summary of Invention

### Technical Problem

An object of the present invention is to provide a means to achieve the storage stability of a lactase solution.

### Solution to Problem

The present inventors attempted to add sugars into a lactase solution based on the techniques according to Patent Literatures 1 and 2. However, the present inventors obtained the findings that the sufficient storage stability of a lactase solution cannot be achieved by simply adding sugars. The present inventors found that by limiting the amount of the sugars to a specific amount, and by setting the amount of reducing sugar to a predetermined value or less, the storage stability of a lactase solution can be achieved, and thus has completed the present invention.

As a result, the present invention provides:
[1] a lactase solution containing sugar in an amount of 0.1 to 50 mg/g on the basis of the total mass of the lactase solution, wherein the amount of reducing sugar contained in the lactase solution is 2.0 mg/g or less on the basis of the total mass of the lactase solution;
[2] the lactase solution according to [1], of which a residual activity after leaving the lactase solution to stand at 50°C for 7 days is 60% or more, wherein the residual activity is determined as a relative value when the activity before the storage is set as 100;
[3] the lactase solution according to [1] or [2], wherein a weight average molecular weight of the sugar is 500 to 2,000,000;
[4] the lactase solution according to any one of [1] to [3], wherein a lactase activity in the solution is 10 to 100,000 NLU/g;
[5] the lactase solution according to anyone of [1] to [4], wherein the lactase solution contains a stabilizer.

### Advantageous Effects of Invention

According to the present invention, the storage stability of lactase as an enzyme is improved. Accordingly, even in a case where there is a time lag between the production of lactase and the addition of the lactase to milk, high lactase activity can be maintained at the time of the addition to milk. In a case where lactase is produced using microorganisms, as described above, the solution containing lactase usually includes substances other than the lactase. In order to remove from the solution the substances other than the lactase (for example, proteins or peptides which have different natures from each other, and various substances with a low molecular weight), various purification processes are required. According to the present invention, by using the amount of the reducing sugar contained in the solution as an indicator, the purification process can be omitted, or the time required for the purification process can be shortened, therefore, the present invention can contribute to the efficiency of the purification process of lactase. The present invention can provide a lactase solution that is excellent in the storage stability, and excellent in the economic efficiency.

### Brief Description of Drawings

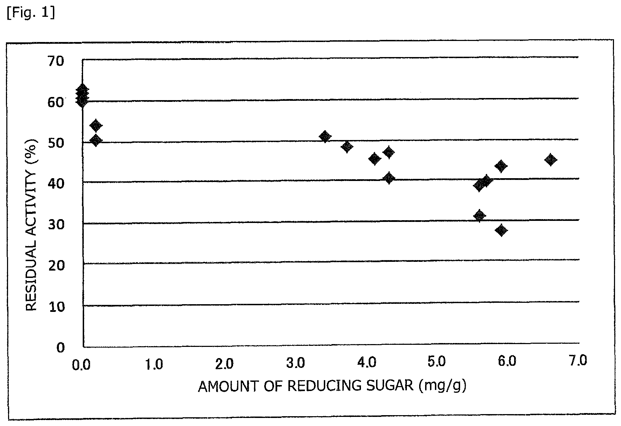

Fig. 1 is a drawing showing the relationship between the amount of reducing sugar in the lactase solution in which sugars has been added and the residual activity of lactase after the lapse of 7 days at 50°C in the lactase solution.

### Description of Embodiments

The lactase solution according to the present invention contains, together with lactase, sugars in an amount to the extent of minimizing the influence on the lactase activity by the reductive sugar-terminal, and optionally contains an additive agent such as a stabilizer. Hereinafter, (1) component of lactase solution, (2) composition of lactase solution, (3) nature of lactase solution (in particular, amount of reducing sugar), (4) production method of lactase solution, and (5) use method and application of lactase solution will be described in this order as follows.

### «Component of lactase solution»

### <Lactase>

### (Kinds of biological raw materials)

Lactase is isolated from an extremely wide range of organisms including microorganisms. The kind of lactase producing microorganisms such as yeast, mold, and bacteria is not particularly limited. Examples of the microorganisms producing lactase include microorganisms belonging to *Kluyveromyces, Aspergillus, Bacillus,* or *Penicillium.* Among them, microorganisms belonging to *Kluyveromyces* are more preferred. Among the microorganisms belonging to *Kluyveromyces, Kluyveromyces fragillis (K. fragillis), Kluyveromyces lactis (K. lactis),* and *Kluyveromyces marxianus (K marxianus)* are preferred, and *K. lactis* is more preferred. Examples of the microorganisms of *Aspergillus* include *Aspergillus oryzae,* or *Aspergillus niger,* and examples of the microorganisms of *Penicillium* include *Penicillium multicolor.*

### (Optimum pH of lactase)

The lactase used in the present invention is neutral lactase or acidic lactase. The neutral lactase and the acidic lactase may be used as a mixture thereof. As the neutral lactase, neutral lactase having the optimum pH of the activity in the neutral region, and having a nature of being deactivated in the acidic region, and further capable of decomposing lactose in the active state is preferred. The neutral lactase having the optimum pH of the activity of 6.0 to 7.5, and the deactivation pH of 4.0 to 6.0 is more preferred. As the acidic lactase, acidic lactase having the optimum pH of the activity in the acidic region, and capable of decomposing lactose in the active state is preferred. The optimum pH of the acidic lactase activity is 3.0 to 5.9.

### <Sugars>

Sugars include all of the monosaccharides, oligosaccharides, and polysaccharides. The kind of the sugar constituting the sugars may be one kind, or may also be two or more kinds thereof. Sugars can have a straight chain structure, a side chain structure, and a ring structure. One sugar of the sugars may have these multiple structures. There is no limitation on the coupling type of the sugar constituting sugars.

### (Reducing sugar)

In a case where sugars contain a large amount of reducing sugar, the storage stability of a lactase solution is largely lowered. Herein, reducing sugar means the one in which a terminal functional group constituting a certain sugar molecule has reducibility. All of monosaccharides are reducing sugar, and some of oligosaccharides and some of polysaccharides are also included in reducing sugar. For example, in a case where sugars are straight-chain polysaccharides, terminals of the sugar have 0, 1, or 2 functional groups showing reducibility. The sugars in which the terminals of the sugar have 1 or 2 functional groups showing reducibility are reducing sugar. In a case where sugars have a side chain structure, the functional groups showing reducibility tend to increase based on its structure than those in straight-chain polysaccharides. From the above definition of reducing sugar, it is preferred to use polysaccharides having a high polymerization degree as the sugars contained in a lactase solution.

The amount of the reducing sugar contained in sugars means a value measured by a DNS method described later. The amount of reducing sugar contained in a lactase solution is required to be 2.0 mg/g or less. The amount of the reducing sugar is preferably 0.5 mg/g or less, and more preferably 0.1 mg/g or less. As the amount of the reducing sugar contained in a lactase solution is increased, the storage stability of a lactase solution tends to be lowered.

### <Polysaccharides>

In the present invention, the term "polysaccharides" is referred to as a saccharide in which seven or more monosaccharides are coupled. For example, starch, cellulose, dextrin, cyclodextrin, pullulan, dextran, arabinoxylan, chitin, chitosan, pectin, inulin, galactan, mannan (galactomannan, glucomannan, and the like), indigestible dextrin, polydextrose, and the like can be mentioned, but there is no particular limitation as long as the polysaccharides are a saccharide capable of being used as a food or drink. These may be used singly or as a mixture of two or more kinds thereof.

Polysaccharides include insoluble, hardly-soluble, and soluble polysaccharides. The insoluble or hardly-soluble polysaccharides easily cause a problem of clogging in a case of being filtered, sterilized or the like by using a carrier of a film or the like for a lactase solution. As the polysaccharides, soluble polysaccharides are preferably used. The soluble polysaccharides mean the polysaccharides having a solubility of 1.5 g or more in 100 g of water at 25°C. Specifically, the soluble polysaccharides are the polysaccharides in which after adding 1.5% by mass of polysaccharides into water and gently stirring the mixture for 24 hours, the absorbance (OD 600 nm) of the mixture solution does not increase by 0.01 or more as compared with that before the addition.

### (Polydextrose)

Polydextrose suitably used for the present invention is produced by polycondensation using glucose as the main raw material in the presence of sorbitol and citric acid, is powdery and water-soluble indigestible polysaccharides, and is widely used as a food material. For example, commercially available polydextrose, "Litesse" (trade name, manufactured by E. I. du Pont de Nemours and Company) that is an improved product of polydextrose, and the like can be mentioned.

### (Pullulan)

Pullulan suitably used for the present invention is a natural polysaccharide obtained by culturing one kind of black yeasts, *Aureobasidium pullulans,* with starch as a raw material, in which maltotriose is linked with regularity by α-1,6 linkage, and is which is a white powder that is tasteless and odorless.

Among them, the most suitable polysaccharide is pullulan, and polydextrose, and among the polydextroses, "Litesse", particularly "Litesse Ultra" is preferred from the viewpoint of the small amount of reducing sugar.

### <Additives>

### (Stabilizer)

In a lactase solution, a stabilizer may be contained. By containing a stabilizer, the lactase activity of a lactase solution can be maintained over a long period of time.

Specific examples of the stabilizer include sorbitol, glycerol, glycerol trimethylolpropane, neopentyl glycol, triethanolamine, glycol, diglycol, triethylene glycol, and glycol, which have a molecular weight of less than 1000. The stabilizer can be contained in a lactase solution alone or by mixing multiple stabilizers thereof.

### (Optional components)

The lactase solution of the present invention may contain various kinds of other components as needed. As the specific example, metal salts contributing to the stabilization of lactase, ascorbic acid, inorganic salts having a buffering action, and the like can be mentioned.

### «Composition of lactase solution»

### <Sugars>

The content of sugars is 0.1 to 50 mg/g on the basis of the total mass of the lactase solution. The content is preferably 0.5 to 50 mg/g, and more preferably 1.0 to 10 mg/g. In a case where the content of sugars is to be less than the lower limit value, the load of the purification process is increased, and it is hard to provide a lactase solution excellent in the economic efficiency. When the content of the sugars exceeds the upper limit value, the storage stability of a lactase solution is easily lowered. Although depending on the kind of sugars, when the viscosity of a lactase solution is increased, a problem in the handleability is easily generated, therefore, there may be some cases where a lactase solution excellent in the economic efficiency cannot be provided.

### <Optional components>

The amount of other optional components to be contained in a lactase solution may be appropriately set.

### <Additives>

### (Stabilizer)

The amount of the stabilizer to be contained in a lactase solution is preferably 10 to 90% by mass, more preferably 20 to 80% by mass, furthermore preferably 30 to 70% by mass, and particularly preferably 40 to 60% by mass. When the amount of the stabilizer is the lower limit value or more, it becomes easy to maintain the lactase activity of a lactase solution over a long period of time. When the amount of the stabilizer exceeds the upper limit value, the viscosity of a lactase solution is increased, and the filtration time is prolonged, and the workability is lowered.

### «Nature of lactase solution»

### <Lactase activity>

The lactase solution of the present invention is desired to have a lactase activity of 10 to 100,000 NLU/g. The expression "NLU" means Neutral Lactase Unit. The measurement method of the activity is, for example, as follows. The activity is measured by hydrolysis of a substrate, o-nitrophenyl-β-galactopyranoside (ONPG) to o-nitrophenyl and galactose. The reaction is terminated by the addition of sodium carbonate. The formed o-nitrophenyl turns yellow in an alkaline medium, and the changes in the absorbance are used to measure the enzyme activity (expressed in NLU/g). This procedure is published on pages 801-802 / lactase (neutral) (β-galactosidase) activity, in the 4th edition of Food Chemical Codex (FCC), U.S.A., effective July 1, 1996.

The lactase activity of a lactase solution is preferably 10 to 250,000 U/mL, more preferably 100 to 120,000 U/mL, and furthermore preferably 2,500 to 60,000 U/mL. The lactase activity can be adjusted by the addition of a solvent, the concentration of lactase solution, or the like.

Various sugars are contained in a lactase solution, and one of the sugars is reducing sugar. As the reducing sugar, for example, reducing sugar derived from a culture medium, reducing sugar derived from the sugars metabolized by lactase-producing microorganisms, reducing sugar modified by the protein metabolized by lactase-producing microorganisms, and the like are contained. As the amount of reducing sugar is increased, it becomes difficult to maintain the lactase activity over a long period of time, therefore, reducing sugar is presumed to decompose the lactase protein, and to give an adverse effect on the stabilization.

### <Method for measuring the amount of reducing sugar>

The amount of the reducing sugar contained in sugars can be measured by the following manner. In a flask in which 100 ml of water (Milli-Q water) has been contained, 1.5 g of the sugars to be measured is added, and then the resultant mixture is stirred at 200 rpm with a magnetic stirrer (manufactured by TIETECH Co., Ltd.), and completely dissolved. The amount of the reducing sugar in each solution is quantified by a DNS method (dinitrosalicylic acid method).

The DNS method is performed by the following procedures. (1) In a test tube, 0.6 mL of a DNS solution (0.7%-3,5-dinitrosalicylic acid, 1.21%-sodium hydroxide, 0.02%-Rochelle salt, 0.57%-phenol, and 0.55%-sodium hydrogen carbonate) is dispensed, and (2) a glucose standard solution (0.1%, and 0.2%) and 0.2 ml of the above-prepared sugars solution are added in the test tube of (1). (3) The resultant mixture is heated and color developed for 5 minutes in a boiling water bath, (4) after water cooling (15°C), 4.2 ml of Milli-Q water is added to the cooled mixture, and the absorbance at a wavelength of 550 nm is measured with a spectrophotometer. The glucose is determined from the standard solution by quantifying the amount of the reducing sugar of the sugars solution as glucose.

### <Stability of lactase activity>

It can be determined by leaving the lactase solution described later to stand under a heating environment for a certain period of time (accelerated stability test) whether or not the lactase activity is maintained for a long period of time. In the present invention, a lactase solution is left to stand under a heating environment at 50°C for 1 to 2 weeks, and then the lactase activity of the lactase solution is measured by a FCC method.

### <Accelerated stability test>

### (Preparation method of samples)

Into a lactase solution (the amount of sugars and reducing sugar is almost 0) in which the lactase activity is adjusted, sugars to be measured are added so that the final concentration is 1.5% by mass, and the resultant mixture is stirred at room temperature so as to be thoroughly dissolved. Subsequently, a sample is obtained through multiple filtration processes. Further, as the sugars to be used for the present tests, soluble polysaccharides are used in all of the tests.

### (Accelerated stability measurement)

The above-prepared sample is dispensed by 10 ml in a 15-ml tube, and stored at 50°C. The lactase activity is measured at the time of 0-day (before storage), 7-day, and 14-day storages, and the residual lactase activity is determined as a relative value when the activity before the storage is set as 100. In the lactase of the present invention, the residual activity at the time of 7-day storage is 60% or more, and preferably 86% or more.

### «Production method of lactase solution»

A specific method for producing the lactase of the present invention includes, for example, (1) an extraction process of the lactase accompanied by disruption of cell walls after yeast culture, and (2) a purification process for removing contaminants and the like, which are derived from the culture, from the extracted lactase. A method for producing the lactase solution of the present invention includes (3) a process of adding sugars (and other additive agents as needed) into the lactase (which may be lactase prepared immediately before the addition or commercially available lactase) as needed, and adjusting the amount of sugars and reducing sugar in the predetermined range, and (4) a process of filtering for the sterilization.

### <Culture and extraction of microorganisms>

The culture of the microorganisms producing lactase is preferably performed at 20 to 40°C for 24 to 180 hours under the condition of pH3 to 10 in a medium containing, for example, lactose and a nitrogen source. In order to collect a lactase solution from the obtained culture, the extraction may be performed, for example, from the recovered cells, or may be performed by using mutant cells or the like that are the cells as discharged outside cells, or may be performed by using the culture medium itself in a case of being cultured in a liquid medium.

### <Purification process>

As the purification method of lactase from a microbial culture, for example, a method of ammonium sulfate fractionation, chromatography such as affinity chromatography, and hydrophobic chromatography, and the like can be mentioned. A variety of substances are contained in a microbial culture, and in order to increase the activity of lactase, these methods are usually used in combination. In addition, the lactase solution can also be pulverized by freeze drying, or spray drying.

### «Use method and application of lactase solution»

The lactase solution of the present invention can be widely used for various kinds of dairy products. Examples of the dairy product include a milk beverage such as milk, fermented milk, ice cream, and milk jam.

A "milk beverage" is a raw material for fermented milk such as yogurt. In the milk beverage, milk beverages before sterilization, and milk beverages after sterilization are also included. The lactase-containing composition can be added before sterilization or after sterilization in accordance with the laws and regulations. Examples of the specific raw material for a milk beverage include water, raw milk, sterilized milk, skim milk, whole milk powder, skimmed milk powder, buttermilk, butter, cream, whey protein concentrate (WPC), whey protein isolate (WPI), α (alpha)-La, and β (beta)-Lg. Gelatin or the like, which are warmed in advance, may be appropriately added. Raw material milk is known, and may be prepared in accordance with a known method. As a raw material for a milk beverage in the present invention, milk is preferably contained. As a raw material for a milk beverage, the raw material made of 100% milk may be used.

The "fermented milk" may be any of yogurt, and the "fermented milk", "dairy product lactic acid bacteria beverage", and "lactic acid bacteria beverage" that are defined by Ministerial Ordinance concerning Milk. In general, plain yogurt is produced by filling a raw material in a container, and then fermenting the raw material (post-fermentation). On the other hand, soft yogurt, and drink yogurt are produced by subjecting fermented milk being fermented to a micronization treatment or a homogenization treatment, and then by filling the resultant product in a container (pre-fermentation). The lactase solution of the present invention can be used for both of the post-fermentation and the pre-fermentation. As a raw material for fermented milk, milk is preferably contained. As a raw material for fermented milk, the raw material made of 100% milk may be used.

In addition, the lactase solution of the present invention can be used in the production of long-life milk. Long-life milk means long-term storage milk. The production process of long-life milk includes a sterilization process and a continuous aseptic packing process, in general, long-life milk is processed by a ultrahigh temperature short time sterilization method at 135 to 150°C for several seconds, and is filled in a paper container in a process capable of aseptic packaging the paper container that has been sterilized with hydrogen peroxide in advance.

### <Application of lactase and the pH profile>

In addition, when considering the application of lactase, the application is roughly classified into two types depending on whether the lactase is neutral lactase or acidic lactase. This is due to the pH profile in application. It can be said that in the neutral pH application, neutral lactase is usually preferred, and acidic lactase is more suitable for the application in an acidic range.

### Examples

The present invention is described in more detail with reference to Examples and Comparative Examples, but the present invention is not in any way limited to these examples.

### <Reference Example 1: Preparation of lactase solution without reducing sugar>

In order to investigate the effect of reducing sugar, a lactase solution not containing reducing sugar was prepared as follows. A liquid medium containing 7% of corn steep liquor and 2% of lactose was autoclaved (pH 5.5 after the sterilization), and then inoculated with *Kluyveromyces lactis* No. 013-2 (ATCC 8585 strain), and the inoculated liquid medium was cultured at 30°C for 24 hours with aeration at 12000 L/min. After completion of the cultivation, the medium was left to stand for 4 hours while cooling, and then the supernatant was removed from the upper part of the tank, and 1500 kg of fungus bodies aggregated and settled to the bottom part of the tank was obtained. Next, among the obtained fungus bodies, 1500 g of the fungus bodies was washed with tap water, and then 80 ml of toluene was added and mixed to the washed fungus bodies, subsequently 1500 ml of 0.05 M phosphate buffer solution (pH7.0) was added to the resultant mixture, and the mixture was stirred and homogenized, and sealed hermetically and left to stand at 30°C for 15 hours for self-digestion.

Into 2500 ml of the supernatant obtained by centrifuging this digested liquid, an equal volume of cold acetone was added, and the resultant mixture was left to stand overnight. The generated precipitate was collected by centrifugation and dissolved in 600 ml of tap water to obtain an enzyme solution. While 600 ml of the enzyme solution before concentration was cooled to 4°C, ammonium sulfate powder was gradually added over 60 minutes to obtain a 50% saturated aqueous solution. The aqueous solution was left to stand (stand still) at 4°C for 80 hours to precipitate lactase, and then the solid-liquid separation was performed by filtration to recover lactase in a solid state. The lactase was redissolved in 600 ml of tap water, and then the resultant mixture was subjected to ultraconcentration. Demineralized water was added to the desalted lactase, and the pH was neutralized to pH 7.5, and then to the neutralized lactase, ammonium sulfate was added so as to be a final concentration of 1 M, and the resultant mixture was desalted to prepare a sample.

The sample after the desalination in a volume of 20 ml was applied to a 20 ml HiPrep phenyl 16/10 column with a diameter of 16 mm, a length of 10 cm, and a linear flow rate of 150 cm/h. The column was equilibrated with 1 M ammonium sulfate in 100 mM Tris with pH 7.5. The column was filled, and then washed with an equilibration buffer at a flow rate of 150 cm/h until reaching the baseline. Elution of the lactase was performed at 150 cm/h under stepwise gradient (100 mM Tris, pH 7.5). After the elution of the lactase, the lactase solution was desalted and concentrated. After the concentration, glycerin was added so that the final concentration is 50%, the mixture was stirred at 200 rpm with a magnetic stirrer (manufactured by TIETECH Co., Ltd.) at room temperature for 18 hours, and a lactase solution (53,000 NLU/g) of Reference Example 1 was obtained. The amount of the sugars and the amount of the reducing sugar, which are contained in the lactase solution of Reference Example 1, were both 0%.

### <Measurement of the amount of reducing sugar>

In accordance with the above-described measurement method of the amount of reducing sugar, for each of the three kinds of Litesse suitable for the present invention, and each of the sugars described in the following Tables 2 and 3, the amount of reducing sugar was measured. The results are shown in Tables 1 and 2.

### [Table 1]

**[Table 1]**

| Name of product | Amount of reducing sugar (mg/g) |
|---|---|
| Litesse II | 1.3 |
| Litesse Powder | 1.2 |
| Litesse Ultra | 0.0 |

### <Accelerated stability test>

### (Preparation of sample)

(1) Each of the sugars in the following Tables 2 and 3 was added to the lactase solution of Reference Example 1 to prepare a sample.

### (Amount of the reducing sugar and residual activity)

Similarly, as to the sugars described in the following Table 2, for the relationship between the amount of reducing sugar measured by the above-described method and the residual activity when the conditions of accelerated stability measurement was set at 50°C and after the lapse of 7 days, the results are shown in Table 2 and Fig. 1.

### [Table 2]

**[Table 2]**

| Trade name | Classification | Amount of reducing sugar (mg/g) | Residual activity (%) |
|---|---|---|---|
| Amicol No. 1 | Starch | 5.7 | 40 |
| Pinedex #1 | Starch decomposition product | 3.7 | 48 |
| Pinedex #2 | Starch decomposition product | 4.3 | 47 |
| Pinedex #4 | Starch decomposition product | 6.6 | 45 |
| Pinedex #100 | Starch decomposition product | 0.2 | 54 |
| H-PDX (H-Pinedex) | Starch decomposition product | 0.0 | 61 |
| MAX 2000N | Starch decomposition product | 3.4 | 51 |
| Pine Fibre | Indigestible dextrin | 4.1 | 46 |
| Celldex SH-20 | Cyclodextrin | 5.9 | 28 |
| Celldex SL-20 | Cyclodextrin | 4.3 | 41 |
| Dixie Pearl SD-20 | Cyclodextrin | 0.0 | 63 |
| Isoelite 40P | Cyclodextrin | 5.6 | 31 |
| Fujioligo #450 | Oligosaccharide | 5.9 | 43 |
| Fujioligo #G67 | Oligosaccharide | 5.6 | 39 |
| Cluster dextrin | - | 0.2 | 50 |
| Pullulan | - | 0.0 | 60 |
| SE 100 | Sugar alcohol | 0.0 | 62 |
| Litesse Ultra | Polydextrose | 0.0 | 63 |

### (Accelerated stability measurement)

For the sample of each of the lactase solutions to which sugars in the following Table 3 were added, by the above-described accelerated stability measurement method, the lactase activity was measured at 50°C and at the time of 0-day, 7-day, and 14-day storages, and at 40°C and at the time of 0-day, 31-day, and 61-day storages, and each residual activity was determined. The results are shown in Table 3.

### [Table 3]

**[Table 3]**

| 50°C | | | |
|---|---|---|---|
| | 0day | 7 days | 14 days |
| Litesse Ultra | 100% | 87% | 46% |
| Pullulan | 100% | 86% | 44% |
| Dixie Pearl | 100% | 81% | 23% |

| 40°C | | | |
|---|---|---|---|
| | 0day | 31 days | 61 days |
| Litesse Ultra | 100% | 88% | 44% |
| Pullulan | 100% | 90% | 40% |
| Dixie Pearl | 100% | 79% | 11% |

### (Results)

It was found that when the amount of the reducing sugar in lactase is 2.0 mg/g or less, the residual activity is at least 60% or more at 50°C and at the time of 7-day storage.

By using the amount of the reducing sugar contained in a lactase solution as an indicator, the purification process of the lactase solution can be omitted, or the time required for the purification process can be shortened, therefore, the present invention can contribute to the efficiency of the purification process of lactase.

## Claims

1. A lactase solution comprising sugar in an amount of 0.1 to 50 mg/g on the basis of the total mass of the lactase solution,
wherein the amount of reducing sugar contained in the lactase solution is 2.0 mg/g or less on the basis of the total mass of the lactase solution.

2. The lactase solution according to claim 1, of which a residual activity after leaving the lactase solution to stand at 50°C for 7 days is 60% or more, wherein the residual activity is determined as a relative value when the activity before the storage is set as 100.

3. The lactase solution according to claim 1 or 2,
wherein a weight average molecular weight of the sugar is 500 to 2,000,000.

4. The lactase solution according to any one of claims 1 to 3, wherein a lactase activity in the solution is 10 to 100,000 NLU/g.

5. The lactase solution according to any one of claims 1 to 4, wherein the lactase solution contains a stabilizer.

## Patentansprüche

1. Laktaselösung, umfassend Zucker in einer Menge von 0,1 bis 50 mg/g, bezogen auf die Gesamtmasse der Laktaselösung, wobei die Menge an reduzierendem Zucker, die in der Laktaselösung enthalten ist, 2,0 mg/g oder weniger beträgt, bezogen auf die Gesamtmasse der Laktaselösung.

2. Laktaselösung gemäß Anspruch 1, wobei die Restaktivität nach dem Stehenlassen der Laktaselösung bei 50°C für 7 Tage 60% oder mehr beträgt, wobei die Restaktivität als ein relativer Wert bestimmt wird, wenn die Aktivität vor der Lagerung auf 100 festgelegt wird.

3. Laktaselösung gemäß Anspruch 1 oder 2, wobei das gewichtsgemittelte Molekulargewicht des Zuckers 500 bis 2.000.000 beträgt.

4. Laktaselösung gemäß einem der Ansprüche 1 bis 3, wobei die Laktaseaktivität in der Lösung 10 bis 100.000 NLU/g beträgt.

5. Laktaselösung gemäß einem der Ansprüche 1 bis 4, wobei die Laktaselösung einen Stabilisator enthält.

## Revendications

1. Solution de lactase comprenant du sucre dans une quantité de 0,1 à 50 mg/g sur la base de la masse totale de la solution de lactase,
dans laquelle la quantité de sucre réducteur contenue dans la solution de lactase est inférieure ou égale à 2,0 mg/g sur la base de la masse totale de la solution de lactase

2. Solution de lactase selon la revendication 1, dont une activité résiduelle après que la solution de lactase a été laissée à reposer à 50 °C pendant 7 jours est supérieure ou égale à 60 %, dans laquelle l'activité résiduelle est déterminée comme une valeur relative lorsque l'activité avant le stockage est fixée à 100.

3. Solution de lactase selon les revendications 1 ou 2,
dans laquelle une masse molaire moyenne en poids du sucre est de 500 à 2 000 000.

4. Solution de lactase selon l'une quelconque des revendications 1 à 3, dans laquelle une activité lactasique dans la solution est de 10 à 100 000 NLU/g.

5. Solution de lactase selon l'une quelconque des revendications 1 à 4, dans laquelle la solution de lactase contient un agent stabilisant.
